# EUROPEAN PATENT SPECIFICATION

(11) **EP 0 925 068 B1**
(45) Date of publication and mention of the grant of the patent: **17.09.2003**
(21) Application number: 98936405.4
(22) Date of filing: 01.07.1998
(51) Int. Cl.: A61K 35/72, A61K 31/05

(54) **USE OF A PHARMACEUTICAL COMPOSITION CONTAINING POLYPHENOLS FROM GRAPES OR FROM WINE, IN PARTICULAR RESVERATROL, AND YEAST EXTRACTS**
VERWENDUNG EINER PHARMAZEUTISCHEN ZUSAMMENSETZUNG DIE POLYPHENOLE AUS WEINTRAUBEN ODER AUS WEIN, INSBESONDERE RESVERATROL, UND HEFEEXTRAKTE ENTHÄLT.
UTILISATION D'UNE COMPOSITION PHARMACEUTIQUE CONTENANT DES POLYPHENOLS DE RAISIN OU DE VIN, EN PARTICULIER, RESVERATROL, ET DES EXTRAITS DE LEVURE

(30) Priority: 02.07.1997 CH 159897
(43) Date of publication of application: 30.06.1999
(73) Proprietor: LABORATORIO ITALIANO BIOCHIMICO FARMACEUTICO LISAPHARMA S.P.A., 22036 Erba (Como) (IT)
(72) Inventor: OSTERWALDER, Nicolò, CH-6900 Lugano (CH)
(74) Representative: Gervasi, Gemma, Dr.
(86) International application number: EP9804065
(87) International publication number: WO99001148

(56) References cited:
- EP-A- 0 773 020
- BERTELLI A A ET AL: "Plasma, urine and tissue levels of trans- and cis- resveratrol (3,4',5-trihydroxystilbene) after short-term or prolonged administration of red wine to rats." INTERNATIONAL JOURNAL OF TISSUE REACTIONS, (1996) 18 (2-3) 67-71, XP002083955
- JANG M. ET AL.: "Cancer chemopreventive Activity of Resveratrol, a Natural Product Derived from Grapes" SCIENCE, vol. 275, no. 5297, 10 January 1997, pages 218-220, XP002083956 cited in the application

## Description

### Field of the invention

The present invention refers to the use of polyphenols from grapes or from wine, in particular 3,5,4'-trihydroxystilbene (resveratrol), combined with yeast, i.e. saccharomyces cerevisiae, for the preparation of oral pharmaceutical compositions for the prevention and treatment of organic conditions connected with the production of free radicals and with tissual anoxial selected from cardiovascular disorders or intestinal biochemism disorders whereby the absorption of polyphenols and the aforementioned biological activity are surprisingly increased.

### State of the Art

The polyphenols contained in grapes and products thereof, and in particular resveratrol, are regarded as mostly responsible for the beneficial effects on a cardiovascular level and on health in general, which, according to recent epidemiological investigations, may be attributed to wine-drinks.

Several studies report that the moderate use of wine lowers the incidence of cardiovascular diseases and the consequent mortality in wine-drinking populations (Seigneur M.J., Appl. Cardiol., 5, 215, 1990; Renaud S., De Longeri M., Lancet, 341, 1103, 1993).

Said beneficial effect is not produced by alcohol, but by other active components present in wine, especially belonging to the class of polyphenols.

Wine and grapes have been found to contain several polyphenols exerting biological activity, e.g. the group of flavonoids, among which catechins and oligomers thereof; flavonols and anthocyanidols, proanthocyanidins, quercitins, as well as their polymers known as tannins. Stilbene derivatives, such as resveratrol, are among non-flavonoid polypenols present in wine.

The polyphenols derived from grapes *(vitis vinifera, vitis rotundifolia, vitis labrusca)* are characterized by the presence of resveratrol in its various forms (cis and trans isomers of 3,5,4'-trihydroxystilbene) and in the form of a glycoside, as well as of anthocyanins, proanthocyanidins, catechins.

While most polyphenols are present in several parts of grapes, resveratrol is especially present in the skin and, like the other polyphenols, is transferred from grapes to wine.

Among the polyphenols derived from grapes and wine, resveratrol has received most attention, due to its varied biological activities. This stilbene, which belongs to the group of phytoalexins, is found in grapes and wine in the form of the cis and trans isomers of resveratrol and in the form of glucoside (cis and trans) which exhibits the same biological activity as non-glucosate resveratrol.

Unlike other polyphenols that are found in several vegetables, resveratrol is especially present in grapes or wine, which represents, therefore, one of the most important sources of resveratrol supply for human beings (Siemann E.H., Creasy L.L., Am. J. Enol. Vitic., 43-52, 1992).

Almost all polyphenols exert antioxidant activity, block the formation of free radicals, which cause several pathologies, reduce oxidative enzymes, and decrease the concentration of lipoperoxides in plasma (Frankel E.N., Kanner Y., German G.B., Parks E., Kinsolle Y.E., Lancet, 341, 454, 1993) (Yuting C.Z., Rongliang Y., Free Radical Biol. Med., 19, 19, 1990).

Said effects are especially produced by resveratrol (Frankel E.N., Waterhouse A.L., Kinsella Y.E., Lancet, 341, 1103, 1993b).

In any case, as reported in several studies, resveratrol is characterized by several biological activities that may have a beneficial effect on health, especially in the prevention of some metabolic alterations' or functional disorders, not only due to oxidative processes.

In fact, evidence has been provided that it can develop an anti-inflammatory and antilipaemic action (Kimura Y., Okuda H:, Arichi S., Biochem. Biophys. Acta, 834, 275, 1985) as well as a thrombocytic antiaggregating action (Bertelli A.A.E., Giovannini L., Int. J. Tiss. Reac., 17, 1, 1995) (Bertelli A.A.E., Giovannini L., Fibrinolysis, 10, Suppl., 1, 1, 1996) (Pace-Ascia K.C.R., Chimica Clinica Acta, 235, 207, 1995).

Resveratrol also reduces vascular damages (Wang Yao-Zhong, Lue Su-Fany, Acta Pharmacologica Sinica, 16, 159, 1995) and carcinogenic processes (Yang M., Cai L., Udeani G.O., Slewug K.V., Thomas C.F., Beecher C.W., Fong H.S., Farusworth N.R., Kinghom D.A., Mehta R., Moon R., Pezzuto Y.M., Science, 275, 218, 1997).

Polyphenols, such as catechins, and anticyanoids exert a complementary and synergic action; for example, the vascular dilatation action is more evident when resveratrol is combined with catechins and anthocyanins (Fitzpatrick D.F., Hirsfield S.L., Coffey R., Am. J. Physiol., 265; Hearth, J. Physiol., 34, H774, 1993).

The vasculo-protective action is, among other actions, a specific activity of anthocyanosides. In fact, vasal permeability and heart activity are protected by anthocyanosides and, in particular, by proanthocyanidins that are the most active of all polyphenols (Ricardo da Silva Y.M., J. of Agric. Food Chemistry, 39, 549, 1991; Hagerman A.E., Butler L.G., J. Biol. Chem., 256, 4494, 1981; Tayeau F., Nivet R., Masqueller Y., Dumas M., Bull. Soc. Chim. Biol., 40, 671, 1958).

Anthocyanosides too, like other polyphenols and, in particular, resveratrol, have shown anticancer activity *in vitro* (Kanei H., Kojima T., Umeda T., Terabe K., Cancer Investigation, 13, 590, 1995), also evidenced through the tumour growth markers (Chen G., Perchellet E.M., Gao X.M., Anticancer Res., 15, 1183, 1995).

As concerns yeasts, it is known that they promote the fermentation causing the transformation of grapes must to wine. The best known yeasts are *leuconostoc* mesenteroides, *leuconostoc oenos kloetrera apiculata, candida* stellata, *pediococcus* pentosaceus, etc. (Martini A.N., Wine Res., 4-165, 1993; Groves S., The Biochemist, 18-13, 1996).

Among said yeasts, the most important and best known is saccharomyces cerevisiae also because of its biological effects. It is rich in proteins, enzymes and vitamins, especially of the vitamin B group, which can be used by the organism for activating glycolytic processes and producing energy bound to ATP formation.

In addition to the vitamin B complex, the yeast proteic fraction includes several enzymes, which may be utilized in important intestinal and organic metabolic activities (Rowland I.R., Drug Metab. Rev., 19, 243, 1988).

In fact, the saccharomyces cerevisiae contains enzymes such as sucrase, lactase, maltase and glucosidase (Harms H.K., Bertele-Hams R.M., Brues-Kleis D., N. Engl. J. Med., 316, 1306, 1987).

The enzymatic action of saccharomyces cerevisiae may be direct as well as indirect, in that it activates the production of enzymes of intestinal microflora (Rowland N., Nugon-Baudon L., Robot S., Intestinal Flora, Immunity, Nutrition and Health, World Rev. Nutr. Diet., Symposium A.P., Corzing T., Rérat A., Eds., Basel,

Karger 174, 123, 1933).

Given the interesting biological properties of polyphenols from grapes and wine, it is important to find pharmaceutical compositions wherein such properties are improved.

The present invention addresses to the problem of obtaining oral pharmaceutical composition of polyphenols with improved biological activity.

### Summary

The Applicant has unexpectedly found that when using a combination of grapes and wine polyphenols, in particular resveratrol, with yeast, i.e. saccharomyces cerevisiae, in the preparation of pharmaceutical composition, the pharmaceutical compositions thus obtained exhibit an improved absorbability when administered by the oral way and a synergic action for the prevention and treatment of organic conditions connected with the production of free radicals and with tissual anoxia selected from cardiovascular disorders or intestinal biochemism disorders.

### Detailed description of the invention

The Applicant has in particular found that polyphenols, when administered in combination with saccharomyces cerevisiae, has a higher bioavailability and a greater biological effect. This was unexpectedly proved by the tests conducted, which are at the basis of the claimed composition.

Polyphenols combined with yeast exert a noticeable synergic effect for absorption and the metabolic and pharmacodynamic actions. The synergic effect cannot be estimated simply by summing up the effects of the single components of the combination. The absorption of polyphenols, in particular of resveratrol, is consideably and unexpectedly favoured when said products are used in combination with the yeast saccharomyces cerevisiae. This is an excellent result considering that polyphenols, in particular resveratrol, when administered per *os,* are scarcely absorbed by the intestine (Bertelli A.A.S., Giovannini L., Stradi R., Bertelli A., Tillement J.P., Int. J. Tiss. Reac., 18, 67, 1996).

Out of the several polyphenols, the composition particularly contains the polyphenols that are present in grapes *(vitis vinifera)* and products thereof, e.g. wine, and more particularly the biologically most active polyphenols, e.g. anthocyanins, catechins, a phytoalexin such as resveratrol, and yeast.

The yeast is one of the yeasts involved in the various steps of the fermentation processes for the transformation of grapes into wine; in particular it is saccharomyces cerevisiae, preferably deprived of bitter substances, in the living and freeze-dried form and in the dried form.

Therefore, the pharmaceutical compositions thus prepared consist of an extract in the powdery or granular or other forms, obtained by drying or by other treatments of grapes or of the residue remaining after pressing of grapes (pomace) or of other wine components, among which wine itself or grape skins or seeds, whereto a dry powder or an extract of yeast, in particular saccharomyces cerevisiae, is combined.

In alternative the compositions according to the invention may comprise a combination of yeast with one or more polyphenols, e.g. resveratrol, wich have been previously isolated from grapes or wine.

The compositions may further contain vitamins, mineral salts and pharmaceutically acceptable excipients.

Preferably the pharmaceutical compositions have a polyphenols/yeast extract ratio ranging from 1:1 to 1:10,000.

The compositions may be solid, semisolid or liquid, and may be used for treating all pathologies responsive to polyphenol therapy. Examples of these conditions are tissue anoxia, cardiovascular disorders, intestinal biochemism disorders, senescence, free radical damages.

It is a further feature of the present invention the use of a combination of polyphenols of grapes and wine, in particular resveratrol, with yeasts, for the manufacture of a medicament having improved polyphenol absorption.

The present invention is further illustrated by the following examples.

### Experimental part

### 1. Product characterization

The compositions according to the present invention was characterized by analysis of the total polyphenols present therein, according to the method developed by Folin-Ciocalteu, modified by Teissedre (Teissedre P.L., Bull. O.l.V., 781-251, 1996). Characterization of 3,5,4'-trihydroxystilbene (resveratrol) was carried out by HPLC-MS according to the method of Lamuela-Raventos (Lamuela-Raventos R.M., J. Agric. Food Chem., 43-283, 1995).

The other polyphenols were identified by HPLC via colourimetric electrode (Achilli G., J. Chromatography, 632-111, 1993).

### 2. Toxicology and pharmacology

The low toxicity and good tolerability of polyphenols, in particular resveratrol, as well as yeasts, e.g. saccharomyces cerevisiae, are well known.

In the tests conducted to estimate the LD₅₀ of said products, the mouse and the rat were administered a mixture of polyphenols or resveratrol alone or dry yeast or a combination of said substances.

The mixture of polyphenols, without resveratrol, was characterized by the presence of catechin (100 mg), epicatechin (25 mg), gallic acid (85 mg), quercitin (12 mg), caffeic acid (25 mg), rutin (20 mg) in a proportion analogous to that present in freeze-dried dealcoholized red wine (Teissedre P.L. and Watherhouse A.L., Bull. O. I. V., 781, 1996).

The resveratrol used was available from Sigma Chemical Co. (St. Louis, MO).

In these tests, no death or toxic effect were ascertained in the treated animals even after administration of 0.5 g/kg polyphenols or 0.25 g/Kg resveratrol or 5 g/kg dry yeast.

Furthermore, no toxic effect was produced by the mixture of the three components administered at the abovesaid doses.

Chronic toxicity tests conducted on the rat administered with the pharmaceutical composition, containing a mixture of 0.20 g/kg polyphenols, 0.1 g/kg resveratrol, and 1 g/kg dry yeast or twice said doses, did not show any sign of toxicity or intolerance in respect of body growth or of various hemochromocytometric or hematochemical parameters.

### 3. Absorbability of resveratrol administered per os

Tests were conducted on the absorbability of resveratrol, administered *per os* either alone or in combination with a mixture of polyphenols or with the dry yeast saccharomyces cerevisiae.

The tested animals were male Wistar rats averagely weighing 300 g each. Products administration was performed *per os* by gastric probing.

Resveratrol concentrations were determined in the plasma of rats at different times from administration, with HPLC-UV-Vis HPLC-MS apparatus with sensitivity limits of 1 ng/ml, according to the methods described by Bertelli et *al.* (Bertelli A.A.E., Giovannini L., Stradi R., Bertelli A., Tillement J.P., Int. J. Tissue Reac. XVII (2/3), 67-71, 1996).

In the various tests, resveratrol was administered either alone at doses of 200 and 400 µg/kg or in combination with 10 mg/Kg of a mixture of polyphenols or in combination with yeast (1-2 g/kg).

Resveratrol concentrations in plasma were determined ½ h, 1 h, and 2 h after products administration (Table 1).

It was found that the administration of resveratrol in combination with yeast resulted in a considerably higher concentration in plasma in comparison with that obtained with resveratrol alone. In fact, as shown in Table 1, the average resveratrol concentration in plasma 1 h after administration was 26 ng/ml, whereas its concentration was almost doubled when it was administered in combination with yeast (40 ng/ml).

The increased concentration induced by yeast was apparent also % h after administration and said concentration levels did not decrease with elapsed time. In fact, the values observed half an hour after administration of resveratrol alone equalled 25 ng/ml, of resveratrol combined with yeast equalled 40 ng/ml. After 1 h, the values were of 35 ng/ml and, respectively, 59 ng/ml.

Therefore, the results obtained show that yeast increases resveratrol absorption. Said increase is also evident when yeast is combined with dehydrated and dried grapes powder containing resveratrol and with a dry dealcoholized concentrated red wine extract, containing a known quantity of resveratrol.

As shown in Table 2 for resveratrol contained in dehydrated and dried grapes powder the average concentration in plasma 1 h after administration was 8 ng/ml, whereas its concentration was almost doubled when it was administered in combination with yeast (14 ng/ml).

The same results were obtained with the dry dealcoholized concentrated red wine estract containing resveratrol, which average concentration in plasma 1 h after administration was respectively 18 ng/ml and 30 ng/ml when it was administered alone or in combination with saccharomyces cerevisiae.

Analogous tests on the effect of saccharomyces cerevisiae on resveratrol absorption were conducted using catechin as a polyphenol component, present in appreciable concentrations in grapes and wine.

Said tests were performed on chickens fed with a diet deprived of catechin, according to Teissedre P.L. *et al.* (Bulletin de l'O.I.V., 781, 1996); catechin was then dosed according to the method developed by Jaworski A.W. *et al.* (J. Agric. Food Chem., 35, 257, 1987) and modified by Lamuela Raventos et al. (Am. J. Enol. Vitic., 45, 1, 1994).

Chickens were administered 400 mg/kg catechin alone or in combination with 2 g/kg yeast, saccharomyces cerevisiae, every day for 10 consecutive days. The catechin plasma levels obtained with catechins combined with saccharomyces cerevisiae were approx. twice those obtained in the absence of yeast (2.58±0.34 nmol/l in animals treated with catechin alone and 4.48±4.2 nmol/l in animals treated with catechin plus yeast). Evidence is thus provided that saccharomyces cerevisiae can increase the absorption of polyphenol catechin.

**Table 1 -**

| Resveratrol average concentrations in the blood of the rat (10 rats per group) after oral administration of resveratrol alone (200 µg/kg and 400 µg/kg) or in combination with a mixture of polyphenols (10 mg/kg) or in combination with yeast, saccharomyces cerevisiae (1 g/kg and 2 g/kg) | | | | |
|---|---|---|---|---|
| Treatment | Dose | ng/ml plasma | | |
| | | 30 | 60 | 120 |
| | | minutes after administration | | |
| Resveratrol | 200 µg/kg | 18±02 | 26±2.1 | 12±0.9 |
| Resveratrol | 400 µg/kg | 25±1.8 | 35±2.9 | 15±1.1 |
| Polyphenols + Resveratrol | 10 mg/kg 200 µg/kg | 20±1.5 | 28±2.5 | 15±1.7 |
| Polyphenols + Resveratrol | 10 mg/kg 400 µg/kg | 27±2.2 | 37±3.2 | 18±1.9 |
| Yeast + Resveratrol | 1 g/kg 200 µg/kg | 22±2.8 | 33±2.7 | 17±0.6 |
| Yeast + Resveratrol | 2g/kg 200 µg/kg | 30±2.1 | 40±3.3 | 24±1.9 |
| Yeast + Resveratrol | 1 g/kg 400 µg/kg | 36±2.9 | 50±5.5 | 30±3.1 |
| Yeast + Resveratrol | 2g/kg 400 µg/kg | 40±4.1 | 59±6.7 | 34±2.9 |

**Table 2 -**

| Resveratrol average concentrations in the blood of the rat (10 rats per group) after oral administration of dehydrated and freeze-dried grapes (pomace) powder or of dry dealcoholized concentrated wine extract containing resveratrol, alone or in combination with saccharomyces cerevisiae (2 g/kg) | | | | |
|---|---|---|---|---|
| Treatment | Dose | ng/ml plasma | | |
| | | 30 | 60 | 120 |
| | | minutes after administration | | |
| Dehydrated and freeze-dried grapes powder (resveratrol concentration = 150 mg/100 g) | 2 g/kg | 5±0.2 | 8±0.5 | 4±0.2 |
| | | | | |
| Dry dealcoholized concentrated wine extract (resveratrol concentration = 500 mg/100 g) | 2 g/kg | 9±1.1 | 18±1.9 | 6±0.3 |
| | | | | |
| Dehydrated and freeze-dried grapes powder (resveratrol concentration =150mg/100g) | 2 g/Kg | | | |
| | + | | | |
| | | | | |
| Yeast | 2 g/Kg | 8±0m9 | 14±1m6 | 6±0.8 |
| | | | | |
| Dry dealcoholized concentrated wine extact (resveratrol concentration = 500 mg/100 g) | 2g/Kg | | | |
| | + | | | |
| | | | | |
| Yeast | 2 g/Kg | 12±1.2 | 30±2.2 | |
| 9±1.1 | | | | |

### 4. Pharmacological activity

Rats were injected intravenous 1 unit/kg pitressin spontaneously to induce myocardial anoxia.

The coronaric spasm resulted in a decreased myocardial oxygenation, while the electrocardiogram showed a typical asphyxial T curve.

In these tests, male Wistar rats averagely weighing 300 g each, before being injected pitressin, were administered resveratrol alone, or resveratrol in combination with the mixture of polyphenols or in combination with yeast, or the combination of the three groups of products. Said products were administered every day for seven consecutive days before pitressin injection. The doses administered were as follows: 100 µg/kg resveratrol, 100 mg/kg polyphenols, and 1 g/kg yeast. The combination consisted of 100 µg/kg resveratrol with 100 mg/kg polyphenols or with 1 g/kg yeast or of resveratrol with polyphenols and yeast at the doses indicated above.

The same tests were also carried out after administration, for seven consecutive days, of dehydrated and freeze-dried grapes powder with resveratrol concentration of 150 µg/100 g or of a dry dealcoholized concentrated wine extract with resveratrol concentration of 500 µg/100g.

Tests were conducted by administering said products alone or combined with 2 g/kg yeast.

The pitressin injected caused the appearance of the asphyxial T wave in the group of animals treated with polyphenols and with yeast; its appearance was reduced by 30% in animals treated with resveratrol, by 40% in animals treated with resveratrol and polyphenols, and by 80% in animals treated with resveratrol and yeast.

The appearance of the asphyxial T wave was completely hindered in animals treated with resveratrol combined with polyphenols and yeast.

Therefore, said tests suggest that yeast potentiates the antianoxic properties of resveratrol.

Analogous positive results were obtained from tests carried out with dehydrated and freeze-dried grapes powder and dry dealcoholized concentrated wine extract.

In these tests too, the appearance of the asphyxial T wave was reduced by more than 50% in respect of controls.

It is a feature of the present invention the use of a combination of polyphenols from grapes or from wine, in particular resveratrol, with yeasts, for the manufacture of a medicament having improved polyphenol absorption for the prevention and treatment of organic conditions as defined in claim 1.

Preferably the used pharmaceutical compositions have a polyphenols/yeast extract ratio ranging from 1:1 to 1:10,000. The compositions may be solid, semisolid or liquid.

### 5. Preparation of pharmaceutical compositions

The various compositions used in the present invention were prepared by different routes depending on the formulation considered. The concentrated wine extract was generally obtained as follows: wine was dealcoholized by distillation and concentrated in a rotary evaporator under vacuum at a temperature of 20 to 30°C. Final drying was carried out in an air stream at a temperature of 30 to 41°C.

Grape-seed powder was prepared once seeds had been separated from pomace by sieving. Grape-seeds were washed with water, dried at room temperature, subjected to oil extraction by pressure or with solvents (hexane), and finely ground to obtain a brownish-grey dry powder.

The grape skins or pomace powder was prepared by drying the starting product in a hot air stream (30 to 40°C). The resulting product was then ground and triturated to a brownish-red powder, which may be used as is or in the freeze-dried form.

For the preparation of tablets resveratrol was mixed with saccharomyces cerevisiae, and the powder of grapes or of seeds or the dry dealcoholized wine extract was mixed with the same yeast (saccharomyces cerevisiae), in appropriate proportions after addition of microcristalline cellulose, in a suitable powder mixer. The powder was homogenized by causing it to pass through an appropriate stainless steel screen. An additional quantity of micronized cellulose, and magnesium stearate were added; the resulting powder was mixed until obtaining a homogeneous dispersion. The powder was compressed using a tablet machine provided with flat or hollow stainless steel punches. Convex tablets could be filmed with gastrosoluble polymers.

For the preparation of capsules resveratrol was mixed in a suitable powder mixer with saccharomyces cerevisiae and with the powder of grapes or of seeds or the dry dealcoholized wine extract, in appropriate proportions, added with powdery lactose, talc and magnesium. Once the blend had become homogeneous, it was filled into hard gelatine capsules.

Some examples of the used pharmaceutical compositions formulated according to the traditional pharmaceutical technique, wherein the polyphenols derived from grapes or wine may be standardized by the present concentrations of resveratrol, anthocyanins and proanthocyanidins, catechins or other polyphenolic components, are as follows:

| | | |
|---|---|---|
| Grapes or wine polyphenols | 100 mg | 1 |
| Resveratrol | 0.5 mg | |
| Yeast (saccharomyces cerevisiae) | 100 mg | |
| | | |
| Grapes or wine polyphenols | 300 mg | 2 |
| Resveratrol | 5 mg | |
| Yeast (saccharomyces cerevisiae) | 300 mg | |
| | | |
| Dehydrated and freeze-dried grapes powder | 400 mg | 3 |
| Polyphenols | 100 mg | |
| Resveratrol | 1 mg | |
| Yeast (saccharomyces cerevisiae) | 100 mg | |
| | | |
| Dry dealcoholized concentrated wine | | |
| extract | 250 mg | 4 |
| Polyphenols | 50 mg | |
| Proanthocyanidins | 20 mg | |
| Anthocyanosides | 10 mg | |
| Resveratrol | 1 mg | |
| Yeast (saccharomyces cerevisiae) | 200 mg | |
| | | |
| Polyphenolic wine extract | 300 mg | 5 |
| Resveratrol | 0.3 mg | |
| Anthocyanidins | 10 | |
| Yeast (saccharomyces cerevisiae) | 400 mg | |
| | | |
| Dehydrated and dried grapes powder | 200 mg | 6 |
| Polyphenols | 100 mg | |
| Resveratrol | 0.25 µg | |
| Catechins | 2.5 mg | |
| Epicatechins | 2.5 mg | |
| Yeast (saccharomyces cerevisiae) | 200 mg | |
| | | |
| Grapes seeds powder | 100 mg | 7 |
| Resveratrol | 0.1 mg | |
| Proanthocyanidins | 20 mg | |
| Yeast (saccharomyces cerevisiae) | 300 mg | |
| | | |
| Grapes seeds oil | 100 mg | 8 |
| Resveratrol | 0.1 mg | |
| Proanthocyanidins | 20 mg | |
| Yeast (saccharomyces cerevisiae) | 300 mg | |
| | | |
| Grapes or wine polyphenols | 100 mg | 9 |
| Resveratrol | 1 mg | |
| Yeast (saccharomyces cerevisiae) | 200 mg | |
| Vit. B₁ | 1 mg | |
| Vit. B₂ | 1 mg | |
| Vit. B₆ | 3 mg | |
| Pantothenic acid | 3 mg | |
| Vit. E | 2 mg | |
| β-carotene | 6 mg | |
| Selenium | 1 mg | |
| Copper | 1 mg | |
| Zinc | 3 mg | |
| Mgnesium | 5 mg | |

## Claims

1. Use of polyphenols from grapes or from wine in combination with yeast extracts for the preparation of oral pharmaceutical compositions for the prevention and treatment of organic conditions connected with the production of free radicals and with tissual anoxia, selected from cardiovascular disorders or intestinal biochemism disorders, said pharmaceutical compositions being optionally added with vitamins, mineral salts and pharmaceutically acceptable excipients.

2. The use according to claim 1, wherein said polyphenols are chosen from the group consisting of anthocyanins, proanthocyanidins, catechins and phytoalexins.

3. The use according to claim 2, wherein said phytoalexin is 3,5,4'-trihydroxystilbene (resveratrol) in its various isomeric forms (cis or trans) and in the form of cis or trans glycoside, or in the form of esters or other analogous derivatives.

4. The use according to claim 3, wherein resveratrol is present in the form of trans 3,5,4'-trihydroxystilbene or in the form of cis 3,5,4'-trihydroxystilbene.

5. The use according to any of claims 1 through 4, wherein the yeast extract is represented by the yeast saccharomyces cerevisiae in the dry or freeze-dried forms.

6. The use according to claim 5, wherein said yeast saccharomyces cerevisiae is first deprived of its bitter substances.

7. The use according to any of claims 1 through 6, wherein the grapes or wine polyphenols/yeast extracts ratio by wt. ranges from 1:1 to 1:10,000.

8. The use according to any of claims 1 through 7, wherein the ratio by wt. of resveratrol to the other grapes or wine polyphenols ranges from 1:1 to 1:10,000.

9. The use according to any of claims 1 through 8 wherein said oral pharmaceutical composition is in the solid or semisolid or liquid form.

10. The use according to claim 8 wherein said pharmaceutical composition is in the form of capsules, tablets, granules, syrup or vials for oral administration.

## Patentansprüche

1. Gebrauch von Polyphenolen aus Trauben oder aus Wein in Kombination mit Hefeextrakten zur Herstellung einer oralen pharmazeutischen Zusammensetzung für die Prävention und die Behandlung von organischen Zuständen, die mit der Produktion von freien Radikalen und mit Gewebsanoxie in Verbindung stehen. Diese Zustände sind dem Bereich der kardiovaskulären Erkrankungen oder der biochemischen Störungen des Magen-Darmtraktes zuzurechnen, wobei der besagten pharämzeutischen Zusammensetzung optional Vitamine, Mineralsalze und pharmazeutisch akzeptable Trägersubstanzen zugesetzt werden.

2. Der Gebrauch gemäß Anspruch 1, wobei die besagten Polyphenole aus der Gruppe ausgewählt werden, die Anthozyanine, Proanthozyanidine, Katechine und Phytoalexine umfasst.

3. Der Gebrauch gemäß Anspruch 2, wobei es sich bei dem besagten Phytoalexin um 3,5,4'-Trihydroxystilben (Resveratrol) in seinen zahlreichen Isomerformen (cis oder trans) und in der Form von cis- oder trans- Glykosid oder in der Form von Estem oder anderen analogen Derivativen handelt.

4. Der Gebrauch gemäß Anspruch 3, wobei Resveratrol in der Form von trans-3,5,4'-Trihydroxystilben oder in Form von cis-3,5,4'-Trihydroxystilben vorliegt.

5. Der Gebrauch gemäß einem der Ansprüche 1 bis 4, wobei es sich bei dem Hefeextrakt um Hefe von Saccharomyces cerevisiae in getrockneter oder gefriergetrockneter Form handelt.

6. Der Gebrauch gemäß Anspruch 5, wobei die besagte Saccharomyces cerevisiae-Hefe zuerst von ihren bitteren Substanzen befreit wird.

7. Der Gebrauch gemäß den Ansprüchen von 1 bis 6, wobei das Gewichtsverhältnis von den in Trauben oder Wein enthaltenen Polyphenolen/Hefeextrakt im Bereich von 1:1 bis 1: 10.000 liegt.

8. Der Gebrauch gemäß den Ansprüchen von 1 bis 7, wobei das Gewichtsverhältnis von Resveratrol zu den anderen in Trauben oder Wein enthaltenen Polyphenolen im Bereich von 1:1 bis 1: 10.000 liegt.

9. Der Gebrauch gemäß einem der Ansprüche von 1 bis 8, wobei die besagte orale pharmazeutische Zusammensetzung in fester, halbfester oder flüssiger Form vorliegen.

10. Der Gebrauch gemäß dem Anspruch 8, wobei die besagte pharmazeutische Zusammensetzung in der Form von Kapseln, Tabletten, Granulat, Syrup oder Ampullen zur oralen Verabreichung vorliegt.

## Revendications

1. L'utilisation de polyphénols à partir de raisins ou de vins en combinaison avec des extraits de levure pour la préparation de compositions pharmaceutiques orales, pour la prévention et le traitement d'états organiques liés à la production de radicaux libres et d'anoxies tissulaires, choisies parmi les troubles vasculaires ou les troubles intestinaux biochimiques, lesdites compositions pharmaceutiques étant le cas échéant additionnées de vitamines, de sels minéraux et d'excipients pharmaceutiquement acceptables.

2. L'utilisation selon la revendication 1, dans laquelle lesdits polyphénols sont choisis dans le groupe constitué par les anthocyanines, les proanthocyanines, les catéchines et les phytoalexines.

3. L'utilisation selon la revendication 2, dans laquelle ladite phytoalexine est le 3,5,4'-trihydroxystilbène (resveratrol) sous ses diverses formes isomères (cis ou trans) et sous la forme cis ou trans glucoside, ou sous la forme d'esters d'autres dérivés analogues.

4. L'utilisation selon la revendication 3, dans laquelle le resveratrol est présent sous forme de trans-3,5,4'-trihydroxystilbène ou sous forme de cis-3,5,4'-trihydroxystilbéne.

5. L'utilisation selon l'une quelconque des revendications 1 à 4, dans laquelle l'extrait de levure est représenté par la levure Saccharomyces cerevisiae sous ses formes sèches ou séchée à froid.

6. L'utilisation selon la revendication 5, dans laquelle ladite levure Saccharomyces cerevisiae est d'abord traitée pour éliminer ses substances amères.

7. L'utilisation selon l'une quelconque des revendications 1 à 6, dans laquelle le rapport en poids polyphénols de raisin ou de vin / extraits de levure est compris entre 1:1 et 1:10.000.

8. L'utilisation selon l'une quelconque des- revendications 1 à 7, dans laquelle le rapport en poids du resveratrol aux autres polyphénols de raisin ou de vin est compris entre 1:1 et 1:10.000.

9. L'utilisation selon l'une quelconque des revendications 1 à 8, dans laquelle ladite composition pharmaceutique orale est sous forme solide, semi-solide ou liquide.

10. L'utilisation selon la revendication 8, dans laquelle ladite composition pharmaceutique est sous forme de gélules, comprimés, granulés, sirops, ou fioles pour administration orale.
